# EUROPEAN PATENT APPLICATION

(11) **EP 1 020 519 A1**
(43) Date of publication of application: **19.07.2000**
(21) Application number: 99200112.3
(22) Date of filing: 15.01.1999
(51) Int. Cl.: C12N 15/12, C07K 14/705, C12N 15/86, C12N 5/10, A61K 39/00, A61K 31/70, C12N 5/08, A61K 35/14, C07K 16/28, G01N 33/574, G01N 33/577, A61K 39/395, C12Q 1/68

(54) **Minor histocompatibility antigens and their use in the diagnosis and treatment of tumors**

(71) Applicant: Introgene B.V., 2333 AL Leiden (NL)
(72) Inventor: Dolstra, Harmen, 6605 XL Wychen (NL); van de Wiel - van Kemenade, Petronella, 3723 BP Bilthoven (NL); Verlinden, Stefan Frederick Franciscus, 2312 WB Leiden (NL)
(74) Representative: Ottevangers, Sietse Ulbe

(57) **Abstract**

Human minor histocompatibility Ags (mHag) play an important' role in the induction of CTL reactivity against tumours after HLA-identical allogeneic bone marrow transplantation (BMT). As most mHag are not tumour specific but also expressed by normal tissues, anti-tumour reactivity is often associated with life-threatening graft-versus-host disease (GVHD). Here, we describe the use of newly found tumour specific mHag for the development of novel means and methods for the treament of cancer.

In one example we identified a novel mHag, HB-1, that elicits donor-derived CTL reactivity in a B-cell acute lymphoblastic leukemia (B-ALL) patient treated by HLA-matched BMT. We identified the gene encoding the antigenic peptide recognised by a HB-1 specific CTL. Expression of the *HB-1* gene was only observed in B-ALL cells and EBV-transformed B cells. Analysis revealed that the *HB-1* gene is polymorphic. The restricted expression of the polymorphic HB-1 Ag by B-ALL cells was used in one example of the invention to generate HB-1 specific CTL in vitro using peptide-loaded dendritic cells. HB-1 specific CTL against B-ALL were used to combat B-ALL and were shown to at least in part reduce the growth of said leukemia without evoking GVHD.

### Annex to the description

SEQUENCE LISTING

<110> Introgene B.V.

<120> Detection and treatment of tumours

<130> p48402ep00

<140> 99200112.3
<141> 1999-01-15

<160> 17

<170> PatentIn Ver. 2.1

<210> 1
<211> 10
<212> PRT
<213> Homo sapiens

<220>
<221> PEPTIDE
<222> (1)..(10)

<400> 1
Glu Glu Lys Arg Gly Ser Leu His Val Trp
  1               5                  10

<210> 2
<211> 10
<212> PRT
<213> Homo sapiens

<220>
<221> PEPTIDE
<222> (1)..(10)

<400> 2
Glu Glu Lys Arg Gly Ser Leu Tyr Val Trp
  1               5                  10

<210> 3
<211> 22
<212> DNA
<213> Homo sapiens

<220>
<221> primer_bind
<222> (1)..(22)
<223> /Note = "primer HLA-5UTR"

<400> 3
gactcagaat tctccccaga cg                                          22

<210> 4
<211> 19
<212> DNA
<213> Homo sapiens

<220>
<221> primer_bind
<222> (1)..(19)
<223> /Note = "primer HLA-3UTR"

<400> 4
ctcagtccct cacaaggca                                                    19


<210> 5
<211> 20
<212> DNA
<213> Homo sapiens

<220>
<221> primer_bind
<222> (1)..(20)
<223> /Note = "primer HB1-F5, sense"

<400> 5
gagccttctg acctcacatc                                                   20


<210> 6
<211> 21
<212> DNA
<213> Homo sapiens

<220>
<221> primer_bind
<222> (1)..(21)
<223> /Note = "primer HB1-GSP3, antisense"

<400> 6
ttgtccctgc tcatccacac c                                                 21


<210> 7
<211> 30
<212> DNA
<213> Homo sapiens

<220>
<221> primer_bind
<222> (1)..(30)
<223> /Note = " HB1-probe, antisense"

<400> 7
tcccttctcg acacggagtc tatgtgtagt                                        30


<210> 8
<211> 19
<212> DNA
<213> Homo sapiens

<220>
<221> primer_bind
<222> (1)..(19)
<223> /Note = "primer Pbgd-R"

<400> 8
gggtacccac gcgaatcac                                                    19

<210> 9
<211> 16
<212> DNA
<213> Homo sapiens

<220>
<221> primer_bind
<222> (1)..(16)
<223> /Note = "primer Pbgd-F"

<400> 9
ggcaatgcgg ctgcaa                                                    16


<210> 10
<211> 27
<212> DNA
<213> Homo sapiens

<220>
<221> primer_bind
<222> (1)..(27)
<223> /Note = " Pbgd-probe"

<400> 10
ctcatctttg ggctgttttc ttccgcc                                        27


<210> 11
<211> 9
<212> PRT
<213> Homo sapiens

<220>
<221> PEPTIDE
<222> (1)..(9)

<400> 11
Glu Glu Lys Arg Gly Ser Leu His Val
1               5


<210> 12
<211> 10
<212> PRT
<213> Homo sapiens

<220>
<221> PEPTIDE
<222> (1)..(10)
<223> /Note = "EBNA3C, Epstein Barr virus"

<400> 12
Glu Glu Asn Leu Leu Asp Phe Val Arg Phe
1               5                   10


<210> 13
<211> 397
<212> DNA
<213> Homo sapiens

<220>
<221> CDS
<222> (108)..(233)
<223> /Note = "sequence of HB-1 cDNA"

<400> 13
ccacatccca ggaggccgag gcggcttgcc ccgcatctca gaagccgggc aggccctgag 60

ccttctgacc tcacatcctc tgccacacca cagtggagaa accagaa ctg gag gag 116
Leu Glu Glu
1

cag cca gaa tgc aga gaa gaa aaa aga ggt tct ctg cat gtt tgg aag 164
Gln Pro Glu Cys Arg Glu Glu Lys Arg Gly Ser Leu His Val Trp Lys
    5                   10                  15

tcg gaa ttg gtt gaa gtt gaa gat gat gtg tat ctg agg cac agc tct 212
Ser Glu Leu Val Glu Val Glu Asp Asp Val Tyr Leu Arg His Ser Ser
20                  25                  30                  35

tcc ctg act tat agg ctt tga cactgctgtt gaggtttgac tcgaagccca 263
Ser Leu Thr Tyr Arg Leu
                40

gagttttggt gtggatgagc agggacaaat tgctgagcat gaagaagagt aaaattaagc 323

aagtggaaca tatgcccttt gcctctgctc tgcacagtga aatgaaaagt caacctttga 383

aaaaaaaaaa aaaa 397

<210> 14
<211> 41
<212> PRT
<213> Homo sapiens

<400> 14
Leu Glu Glu Gln Pro Glu Cys Arg Glu Glu Lys Arg Gly Ser Leu His
1               5                   10                  15

Val Trp Lys Ser Glu Leu Val Glu Val Glu Asp Asp Val Tyr Leu Arg
            20                  25                  30

His Ser Ser Ser Leu Thr Tyr Arg Leu
        35                  40

<210> 15
<211> 41
<212> PRT
<213> Homo sapiens

<220>
<221> PEPTIDE
<222> (1)..(41)

<400> 15
Met Glu Glu Gln Pro Glu Cys Arg Glu Glu Lys Arg Gly Ser Leu His
1               5                   10                  15

Val Trp Lys Ser Glu Leu Val Glu Val Glu Asp Asp Val Tyr Leu Arg
            20                  25                  30

His Ser Ser Ser Leu Thr Tyr Arg Leu
        35                  40

<210> 16
<211> 30
<212> DNA
<213> Homo sapiens

<220>
<221> misc_feature
<222> (1)..(30)
<223> /Note = "one of the alternative sequences for a
      region of the HB1-gene"

<400> 16
gaagaaaaaa gaggttctct gcatgtttgg                                30

<210> 17
<211> 30
<212> DNA
<213> Homo sapiens

<220>
<221> misc_feature
<222> (1)..(30)
<223> /Note = "one of the alternative sequences for a
      region of the HB1-gene"

<400> 17
gaagaaaaaa gaggttctct gtatgtttgg                                30

## Description

### FIELD OF THE INVENTION

The invention lies in the field of medicine. More particularly, the invention lies in the field of tumour diagnosis, treatment and the development of medicaments for the treatment of tumours.

### BACKGROUND OF THE INVENTION

Transplantation with HLA-identical sibling bone marrow is successfully used to treat patients with leukemia. The therapeutic effect can be partly contributed to the elimination of residual host leukemic cells by donor-derived T cells, termed the graft-versus-leukemia (GVL)¹ reactivity (1). However, this donor-derived T cell reactivity generally causes graft-versus-host disease (GVHD), a life-threatening complication in allogeneic bone marrow transplantation (BMT) (1). The ability of donor-derived CTL to recognise host minor histocompatibility Ags (mHag) as foreign peptides plays an important role in both GVHD and GVL reactivity (2-4). mHag are HLA-associated peptides generated from polymorphic regions of proteins present in the target cells (5-7). Interestingly, besides mHag with ubiquitous tissue distribution, mHag have also been characterised with expression restricted to hematopoietic cells or leukemic cells (2,8-11).

Although allogeneic BMT may cure many patients with leukemia, relapse of the tumour occurs in a significant number of patients indicating that in these patients not all tumour cells are sufficiently eliminated or suppressed. Therefore, there is a continuous search for additional and more specific immunotherapeutic strategies in the treatment of leukemia in order to eliminate leukemic cells without inducing GVHD.

### SUMMARY OF THE INVENTION

In the present invention nucleotide sequences encoding minor histocompatibility antigens essentially specifically expressed on tumour cells were identified, cloned and coding regions were identified. The present invention utilises antigens essentially specifically expressed on tumour cells for the development of means and methods for the diagnosis and treatment of tumours.

### DETAILED DESCRIPTION OF THE INVENTION

In one aspect the invention utilises minor histocompatibility antigens for the selection of tumour specific antigens for directing, promoting or enhancing or a combination thereof, an immune response against tumour cells.

In one aspect the invention provides a tumour specific antigen or an immunogenic part, derivative or analogue of said antigen, which is a minor histocompatibility antigen. Preferably said antigen is essentially specific for leukemia or lymphoma, preferably B-cell leukemia, more preferably B cell acute lymphoblastic leukemia.

In one aspect the invention provides a tumour specific antigen of the invention wherein said antigen is a polymorphic antigen.

Preferably a tumour specific antigen of the invention is a human antigen.

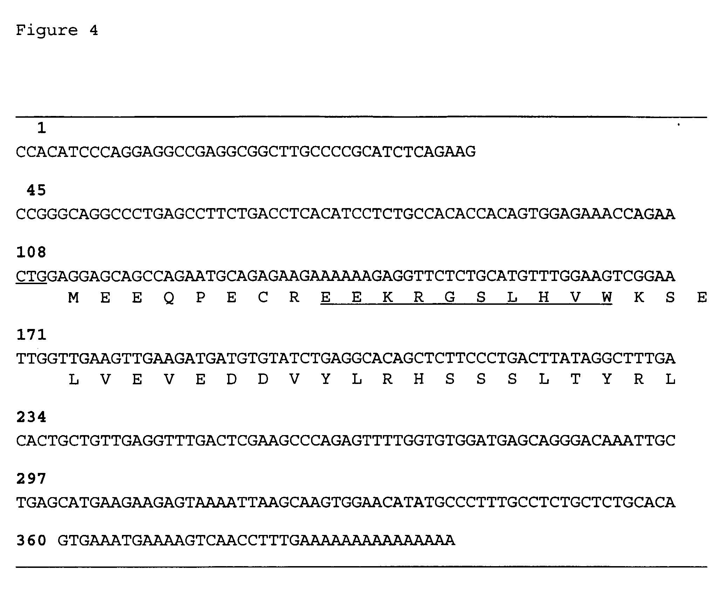

In one embodiment a tumour specific antigen of the invention comprises an amino-acid sequence as depicted in figure 4 or an immunogenic part, derivative or analogue thereof. In one embodiment the invention provides a peptide comprising an immunogenic part of a tumour specific antigen according to the invention. Preferably said peptide is capable of associating with at least one major histocompatibility complex 1 molecule (MHC-I) and/or complex II (MHC-II) molecule. In one embodiment said peptide comprises the amino-acid sequence EEKRGSLHVW and/or the amino-acid sequence EEKRGSLYVW, or a functional part, derivative or analogue thereof.

In one embodiment the invention provides a nucleic acid encoding an antigen or a peptide of the invention. In a preferred embodiment the invention provides a nucleic acid comprising a nucleic acid sequence depicted in figure 4, or a functional part, derivative or analogue thereof.

In another aspect the invention provides a nucleic acid delivery vehicle comprising at least a nucleic acid encoding at least one antigen or peptide of the invention. In a preferred embodiment said nucleic acid delivery vehicle further comprises a nucleic acid encoding at least one protein capable of modulating an immune response. Such a protein may be a co-stimulatory protein such as CD80, CD86, CD28, CD152, CD40 or CD40 ligand; a cell-adhesion protein; an immune response inhibitory protein; an interleukin; a major histocompatibility complex I protein; or a major histocompatibility complex II protein or other proteins capable of modulating an immune response. An immune response may be modulated through at least in part inhibiting or preventing an immune response and/or at least in part inducing or enhancing an immune response. A nucleic acid delivery vehicle comprising one nucleic acid encoding a protein capable of modulating an immune response may further comprise additional nucleic acids encoding other proteins capable of stimulating on immune response.

In one embodiment the invention provides a nucleic acid delivery vehicle according to the invention wherein said vehicle has at least in part been provided with a tissue tropism for smooth muscle cells hemopoietic stem cells, antigen presenting cells, endothelial cells and/or their progenitors.

In another embodiment the invention provides a nucleic acid delivery vehicle according to the invention, wherein said vehicle has at least in part been deprived of tissue tropism for liver cells.

In a preferred embodiment said changed tissue tropism is provided by at least a tissue tropism determining part of a fiber protein of a group B adenovirus, preferably adenovirus 16.

In one aspect a nucleic acid delivery vehicle of the invention comprises a nucleic acid comprising integration means of an adeno-associated virus nucleic acid. Said integration means comprise at least one adeno-associated virus inverted terminal repeat sequence. Preferably said integration means are comprise at least two adeno-associated virus inverted terminal repeat sequences wherein preferable one adeno-associated virus inverted terminal repeat sequence is present on either side of a nucleic acid sequence of which integration into the host chromosome is desired. Integration of a nucleic acid of the invention into the host cell chromosome of a cell is preferred in cases where said nucleic acids is required to be present in said cell for a prolonged period a time or when said cells are expected to undergo cell division subsequent to delivery of said nucleic acid to said cell.

Said nucleic acid delivery vehicle may be any kind of nucleic acid delivery vehicle including non-viral or viral or a . combination of both types of nucleic acid delivery vehicles. Said nucleic acid'delivery vehicle may even comprise water, in cases where nucleic acid is injected into a body through one or more needles. In a preferred embodiment a nucleic acid delivery vehicle according to the invention is an adenovirus particle, an adeno-associated virus particle or a retrovirus particle. Said adenovirus particle may be derived from any type of adenovirus. Preferably said adenovirus particle is derived from adenovirus 5 and/or adenovirus 16. Said adeno-associated virus particle or integration means may be derived from any type of adeno-associated virus. Preferably said adeno-associated virus particle or integration means are derived from adeno-associated virus 2. Said retrovirus particle may derived from any type of retrovirus. Preferably said retrovirus particle is derived from a murine leukemia virus or a lentivirus, preferably human immunodeficiency virus.

In a preferred embodiment said adenovirus particle comprises an adenovirus vector. Said adenovirus vector may be derived from any type of adenovirus. Preferably said adenovirus vector is derived from adenovirus 5. Said adenovirus vector may at least in part deprived of the capability to express E1-region encoded proteins, preferably through a deletion in the E1-region. Said vector may at least in part be deprived of the capability to express E2A protein, preferably through at least a partial deletion of nucleic acid encoding said E2A protein. Said vector may it least in part be deprived of the capability to express at least one of the E3-region encoded proteins, preferably through at least a partial deletion of nucleic acid encoding at least one of said E3-region encoded proteins. Said vector may at least in part be deprived of the capability to express at least one of the E4-region encoded proteins, preferably E4-Orf3 and/or E4-Orf6, preferably through at least a partial deletion of nucleic acid encoding at least one of said E4-region encoded proteins.

Said vector may also be deprived of the capacity to express a combination of one or more of the proteins encoded by said regions or by said E2A-gene.

In another aspect the invention provides a cell for the production of an adenovirus particle of the invention comprising one or more means for the complementation of at least part of the function of one or more of the proteins said vector has at least in part been deprived the capability of expression of. Preferably said cell is derived from a PER.C6 cell (ECACC deposit number 96022940).

In one aspect the invention provides a method for generating, promoting or modulating an immune response against tumour cells in an individual, comprising administering to said individual a composition comprising at least one antigen of the invention, at least one peptide of the invention, at least one nucleic acid of the invention, at least one nucleic acid delivery vehicle of the invention, or at least one adenovirus particle of the invention, or a combination thereof. Preferably said composition further comprises of a pharmaceutically acceptable carrier or diluent.

In a preferred embodiment of the invention tumour cells are leukemic cells or lymphoma cells, preferably leukemic B cells, more preferably acute lymphoblastic leukemic B cells. In another embodiment the invention provides a method for generating tumour cell specific T lymphocytes, comprising culturing CD4 and/or CDS positive T cells together with stimulator cells, wherein said cells have been provided with at least one peptide of the invention. Preferably said T lymphocytes are cytotoxic T lymphocytes, helper T lymphocytes or both. Said stimulator cells may be any cells capable of presenting a peptide of the invention in the context of major histocompatibility complex I or complex II. Preferably said stimulator cells comprise professional antigen presenting cells, more preferably said target cells comprise dendritic cells.

Preferably said T lymphocytes cells and said stimulator cells are derived from HLA-matched donors, identical twins or the same individual.

In one embodiment the invention provides T lymphocytes capable of recognising cells presenting peptides in the context of MHC class I or class II, encoded by a HB-1 gene, or a functional part, derivative or analogue thereof. In one embodiment the invention provides tumour cell specific T lymphocytes obtainable by a method of the invention. In a preferred embodiment of the invention T-lymphocytes of the invention are provided with a nucleic acid encoding a herpes simplex virus thymidine kinase. In another preferred embodiment T-lymphocytes of the invention are provided with a nucleic acid encoding at least one protein capable of modulating an immune response. Such a protein may be a co-stimulatory protein such as CD80, CD86, CD28, CD152, CD40 or CD40 ligand; a cell-adhesion protein; an immune response inhibitory protein; an interleukin; a major histocompatibility complex I protein; a major histocompatibility complex II protein or other proteins capable of modulating an immune response.

In a preferred embodiment of the invention tumour cell specific T lymphocytes are leukemia or lymphoma specific T lymphocytes, preferably leukemic B cell specific T lymphocytes, more preferably acute lymphoblastic leukemic B cell specific T lymphocytes.

In one aspect the invention provides a method for the treatment of an individual suffering from or at risk of suffering from a tumour comprising administering to said individual T-lymphocytes of the invention. Preferably said T lymphocytes are cytotoxic T lymphocytes or helper T lymphocytes or a combination of both.

In another aspect, the invention provides the use of tumour cell-specific T lymphocytes in a medicament. Preferably said T lymphocytes are cytotoxic T lymphocytes or helper T lymphocytes or a combination of both. Preferably said tumour cell specific T lymphocytes are leukemia or lymphoma specific T lymphocytes, preferably leukemic B cell specific T lymphocytes, more preferably acute lymphoblastic leukemic B cell specific T lymphocytes.

In another aspect the invention provides the use of a minor histocompatibility antigen, derivative or analogue thereof, essentially specific for tumour cells for the generation of tumour specific T lymphocytes.

Preferably said T lymphocytes are leukemia or lymphoma specific T lymphocytes, preferably leukemic B cell specific T lymphocytes, more preferably acute lymphoblastic leukemic B cell specific T lymphocytes. Preferably said T lymphocytes are cytotoxic T lymphocytes or helper T lymphocytes or a combination of both.

In yet another aspect the invention provides an antibody with a specificity for an antigen or a peptide of the invention, or both.

Preferably said antibody is an antibody capable of recognising a peptide of the invention in the context of a major histocompatibility complex I molecule and/or a major histocompatibility complex II molecule. Such an antibody may be obtained by immunising a test animal or a human with a preparation comprising exosomes comprising said peptide in the context of said major histocompatibility complex I molecule and/or said major histocompatibility complex II molecule. Said exosomes are preferably obtained from cells expressing the desired major histocompatibility complex I and/or complex II molecule and producing a suitable amount of exosomes. A preferred cell for the production of exsomes is a dendritic cell. Said major histocompatibility complex I and/or complex II molecule present on said exsomes may be provided with a peptide of the invention through any means suitable for the loading of major histocompatibility complex molecules with peptides. Preferably said major histocompatibility complex I and/or complex II molecule is provided with a peptide of the invention through expression of antigen of the invention in a cell producing said exsomes, or said major histocompatibility complex I and/or complex II molecule is loaded with a peptide of the invention by contacting said exosomes or said cells with a peptide of the invention. An antibody capable of recognising a peptide of the invention may be used for diagnostic purposes, enabling a rapid determination of the presence and the type of minor histocompatibility antigen on a cell, preferably a tumour cell. An antibody capable of recognising a peptide of the invention in the context of MHC-I or MHC-II may be used to rapidly determine the presence and the type of presentation of peptides of the invention in the context of a major histocompatibility complex I or complex II molecule on a cell, preferably a tumour cell, since detection of binding of said antibody gives information on both the presence of said peptide and said MHC-I or said MHC-II molecule.

In one aspect the invention provides the use of an antibody of the invention in an immunoassay for the detection of cells, preferably tumour cells, preferably leukemia cells or lymphoma cells, more preferably, B cell leukemia cells, even more preferably acute lymphoblastic leukemic B cell leukemia cells.

In one aspect the invention provides a method for detecting T lymphocytes of the invention comprising loading labelled tetrameric MHC molecules with a peptide of the invention and contacting said T lymphocytes with said peptide loaded MHC molecules, and detecting said label. Said MHC may be MHC I or MHC II derived.

In another aspect the invention provides a vaccine comprising at least an antibody according to the invention, and a pharmaceutically acceptable carrier or diluent.

In another aspect the invention provides a method for the identification of cells, preferably tumour cells comprising detecting RNA encoding an antigen of the invention in a sample comprising RNA of said cells.

In another aspect the invention provides a method for the identification of cells, preferably tumour cells using an antibody of the invention.

In another aspect the invention provides a kit for the detection of cells, preferably tumour cells comprising at least one antibody of the invention.

In yet another aspect the invention provides a kit for the detection of cells, preferably tumour cells comprising at least two primers and a probe directed to a nucleic acid of the invention.

In yet another aspect the invention provides a cloning vehicle comprising a nucleic acid of the invention.

In yet another aspect the invention provides a cell having been provided with a nucleic acid of the invention. In yet another aspect the invention provides a vaccine comprising antigen presenting cells, preferably dendritic cells, containing MHC-I and/or MHC-II molecules with a peptide of the invention.

In yet another aspect the invention provides a vaccine comprising antigen presenting cells, preferably dendritic cells, containing MHC-I and/or MHC-II molecules with a peptide of the invention, wherein said cells are provided with a nucleic acid by a gene delivery vehicle of the invention, or an adenovirus particle of the invention. In another aspect the invention provides a vaccine comprising a gene delivery vehicle and/or an adenovirus particle of the invention.

In yet another aspect the invention provides the use of at least one antigen, at least one peptide, at least one nucleic acid, at least one nucleic acid delivery vehicle, or at least one adenovirus particle of the invention, or a combination thereof in a medicament.

Antigen presenting cells are for instance dendritic cells, monocytes, macrophages and B cells or a combination of these cells. Progenitors of antigen presenting cells are capable of differentiating into functional antigen presenting cells. A nucleic acid of the invention may further comprise sequences allowing expression of any coding regions encoded by said nucleic acid. Such sequences may include promoters, enhancers, locus control regions inducible or not, polyA signals, splice signals, transcription termination signals, translation signals, internal ribosomal entry site signals and other signals enabling appropriate expression of said coding regions.

In the technical part we have focused our attention on one example i.e. CTL that selectively recognise leukemic but not normal cells. However, the invention is not limited to this example. We isolated from a BMT recipient an HLA-B44 restricted CTL clone recognising a novel mHag, named HB-1, that shows specificity for B-cell acute lymphoblastic leukemia (B-ALL). We identified both a nucleotide sequence encoding a HB-1 Ag, and a polymorphic CTL epitope. Furthermore, we show that expression of the *HB-1 gene* is essentially restricted to B-ALL cells and EBV-transformed B cells.

A minor histocompatibility antigen is defined as a polymorphic antigen with proven immune response reactivity. A minor histocompatibility antigen can be defined as an tumour specific antigen.

### EXAMPLES

### Materials and Methods

*Cell culture.* All cell lines were cultured in IMDM (Gibco BRL, Gaithersburg, MD) supplemented with 10% FCS. CTL clone MP1 was isolated from PBL of leukemia patient MP after HLA-identical BMT and grown in IMDM supplemented with 10% pooled human serum, irradiated EBV transformed-lymphoblastoid cell line (EBV-LCL) of the patient pre-BMT (10⁶/ml), irradiated allogeneic PBMC (10⁶/ml), 100 U/ml IL-2 (Glaxo, Geneva, Switzerland), and 0.4 µg/ml PHA.

*cDNA library construction.* The cDNA library was constructed using the Superscript Plasmid System (Gibco BRL). Total RNA was isolated from EBV-LCL MP and poly(A)⁺ mRNA was prepared by oligo-dT binding (Qiagen, Santa Clarita, CA). mRNA was converted into cDNA using an oligo-dT primer that contains a NotI site at its 5' end. The cDNA was ligated to *Sal*I adapters, digested with *Not*I and ligated into *Sal*I and *Not*I sites of the expression vector pSV-Sport1. E. coli DH10B were electroporated with the recombinant plasmids and clones were selected with ampicillin. This library was divided into 1500 pools of ^{~} 100 cDNA clones. Each pool was amplified for 4 h and plasmid DNA was extracted with Bio-rad miniprep kits (Bio-rad Laboratories, Hercules, CA).

*Isolation of the HLA-B*4403 gene.* Total RNA from EBV-LCL MP was extracted using the Trizol method (Gibco BRL). Reverse transcription was performed on 2 µg of total RNA using an oligo-dT primer and Mo-MuLV reverse transcriptase (Gibco BRL). HLA cDNA was amplified by PCR using 50 pmol HLA-5UTR primer (5'-GACTCAGA(AT)TCTCCCCAGACG-3'), 50 pmol HLA-3UTR primer (5'-CTCAGTCCCTCACAAGGCA-3'), 0.5 mM dNTPs and 2.5 U Taq polymerase as described previously (12). After separation of the PCR product on a 1% agarose gel, the 1.2 kb DNA fragment was isolated and cloned into pCR3 vector by using the TA cloning kit (Invitrogen, San Diego, CA). This cloned *HLA-B*4403* gene was sequenced by the dideoxynucleotide chain termination method.

*Transfection of COS-1 cells and CTL stimulation* assay. Transfections were performed by the lipofection method. Briefly, 1.5 x 10⁴ COS-1 cells were plated in a flat bottomed 96-well plate and incubated for 18 h at 37°C. 200 ng of plasmid pCR3 containing the *HLA-B*4403* gene and ^{~} 400 ng of plasmid pSV-sportl containing a pool of the cDNA library were mixed with 1 µl of lipofectamine (Gibco BRL) in 100 µl IMDM. Transfection mixtures were incubated for 30 min and 50 µl was added in duplicate wells to the COS-1 cells. After 4 h of incubation, 50 µl of IMDM/20% FCS was added. Transfectants were tested for their ability to stimulate the production of IFN-γ by the CTL. Briefly, 3000 CTL were added to the wells containing transfected cells or 3x10⁴ stimulator cells in 200 µl IMDM/10% FCS and 25 U/ml IL-2. After 18 h of incubation at 37°C, 100 µl supernatant was collected and the IFN-γ concentration was determined by ELISA (Endogen, Woburn, MA). *Production of truncated and mutated cDNA constructs.* Deletion and mutation constructs were produced by PCR using specific primers and amplified products were subsequently cloned into vector pCR3 using the unidirectional TA cloning method (Invitrogen).

*HB-1 polymorphism.* Total RNA from cells was extracted using the Trizol method (Gibco BRL). Reverse transcription was performed on 2 µg of total RNA using an oligo-dT primer and Mo-MuLV reverse transcriptase (Gibco BRL). HB-1 cDNA was amplified by PCR using 50 pmol HB1-F5 primer (5'-GAGCCTTCTGACCTCACATC-3'), 50 pmol HB1-GSP3 primer (5'-TTGTCCCTGCTCATCCACACC-3'), 0.5 mM dNTPs and 2.5 U Taq polymerase (Gibco BRL). The PCR was performed for 33 cycles (1 min at 94°C, 1 min 60 °C, and 1 min 72°C). PCR products were digested with *Nla*III to discriminate between *HB-1*^{*H*} and *HB-1*^{*Y*} alleles.

*Peptides and chromium release assay.* Peptides were synthesised with a free carboxy-terminus by Fmoc peptide chemistry using an ABIMED multiple synthesiser. Peptides (>90% pure as indicated by analytical HPLC) were dissolved in DMSO and stored at -20 °C. Chromium release assays were performed as described previously (13). In peptide recognition assays, target cells were preincubated with various concentrations of peptide for 1 h at 37°C in a volume of 100 µl prior to the addition of effector cells. After 4 h of incubation at 37°C, 100 µl supernatant was collected and radioactivity was measured by a gamma counter.

*Taqman™ PCR assay for HB-1 expression.* Total RNA from cells was extracted using the Trizol method (Gibco BRL). Reverse transcription was performed on 2 µg of total RNA using an oligo-dT primer and Mo-MuLV reverse transcriptase (Gibco BRL). Of the total cDNA-volume of 20 µl, one µl was used for each PCR reaction. PCR amplification and real time quantitation analysis were performed using the Taqman™ assay (14,15). The following sequences were used as primers and Taqman™ probes: 5'-GAGCCTTCTGACCTCACATC-3' (HB1-F5, sense, nt 58-77), 5'-TTGTCCCTGCTCATCCACACC-3' (HB1-GSP3, antisense, nt 271-291), 5'-(FAM)-TCCCTTCTCGACACGGAGTCTATGTGTAGT-(TAMRA)-3' (HB1-probe, antisense, nt 188-217), 5'-GGCAATGCGGCTGCAA-3' (Pbgd-F), 5'-GGGTACCCACGCGAATCAC-3' (Pbgd-R), and 5'-(JOE)-CTCATCTTTGGGCTGTTTTCTTCCGCC-(TAMRA)-3' (Pbgd-probe). The reaction mixture contains 1.25 U AmpliTaq Gold (Perkin Elmer/Applied Biosystems), 250 µM dNTP, 15 pmol sense and 15 pmol antisense primer in a total reaction volume of 50 µl. The enzyme was activated by heating for 10 min at 95°C. A two-step PCR procedure of 60 s at 60°C and 15 s at 95°C was applied for 40 cycles. 6 mM MgCl2 and 100 nM probe for the HB-1 PCR, and 5 mM MgCl2 and 100 nM probe for the Pbgd PCR were used. The PCR and Taqman™ analysis were performed in the ABI/PRISM 7700 Sequence Detector System (ABI/PE, Foster City, CA). The system generates a real time amplification plot based upon the normalised fluorescence signal. Subsequently the threshold cycle (C_{T}) is determined, i.e. the fractional cycle number at which the number of amplified target reaches a fixed threshold. The C_{T} is proportional to the initial number of target copies in the sample (14,15). We used the expression of the *Pbgd* gene to normalise the HB-1 expression. *Pbgd* was used as an endogenous reference to correct for differences in the amount of total RNA added to the reaction. The Pbgd primers only allows amplification of cDNA derived from the *Pbgd* housekeeping gene (16,17). HB-1 mRNA expression was quantified by determining calibration functions for HB-1 and Pbgd expression of a reference cell line. Therefore, 2 µg of RNA of the B-ALL cell line KM3 was reverse transcribed into cDNA and serially diluted into water. This cDNA serial dilution was prepared once, stored at -20°C, and used in all tests performed in this study. The linear calibration functions between the C_{T} and the logarithm of the initial starting quantity (N) were C_{T}=-3.44log(N)+25.8 and C_{T}=-3.44log(N)+21.9 for HB-1 and Pbgd, respectively. HB-1 and Pbgd mRNA expression in all test samples were quantified using these calibration functions. At the same level of Pbgd expression the level of HB-1 expression of test samples was determined as a percentage of the HB-1 expression in cell line KM3.

### Results

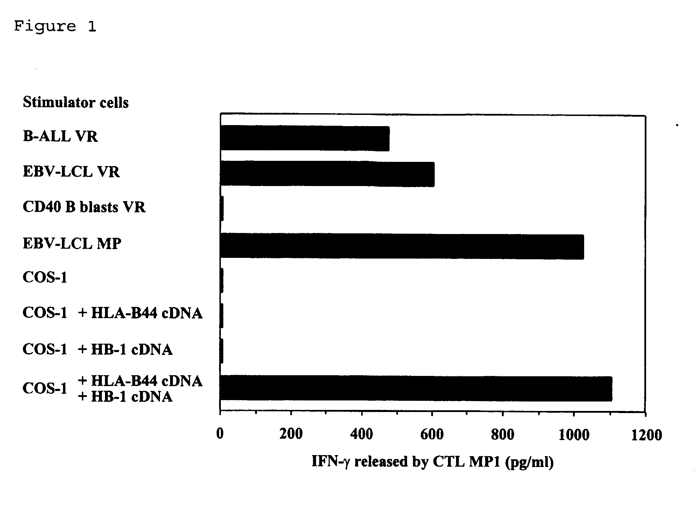

*Identification of a cDNA coding for the HB-1 antigenic peptide.* We generated a cDNA library from an EBV-LCL that expresses the HB-1 Ag since it was efficiently lysed by CTL MP1. Using expression cloning, we isolated from this library a cDNA which upon transfection along with the HLA-B44 cDNA into COS-1 cells stimulated CTL MP1 to release IFN-γ (Fig. 1). The IFN-γ release was as high as that induced by EBV-LCL MP, from which the cDNA library was generated, and even higher than that induced by EBV-LCL as well as B-ALL cells of the HLA-B44, HB-1-positive patient VR. Untransformed CD40-stimulated B cells of patient VR could not stimulate CTL MP1 to release IFN-γ. Transfection of either of the aforementioned cDNAs alone failed to induce the production of IFN-γ by CTL MP1 indicating that the isolated cDNA clone encodes the HB-1 Ag.

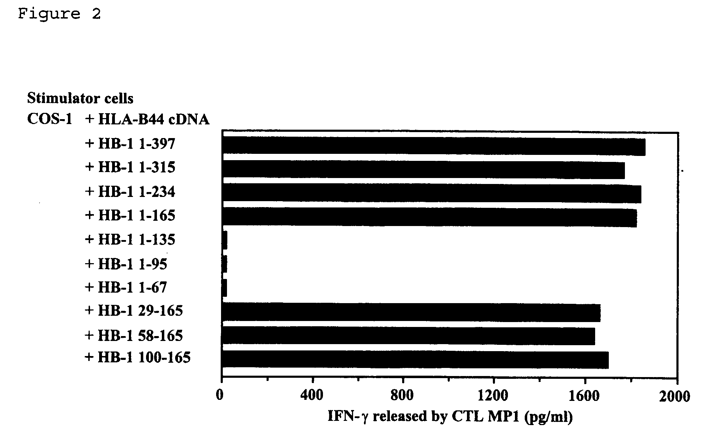

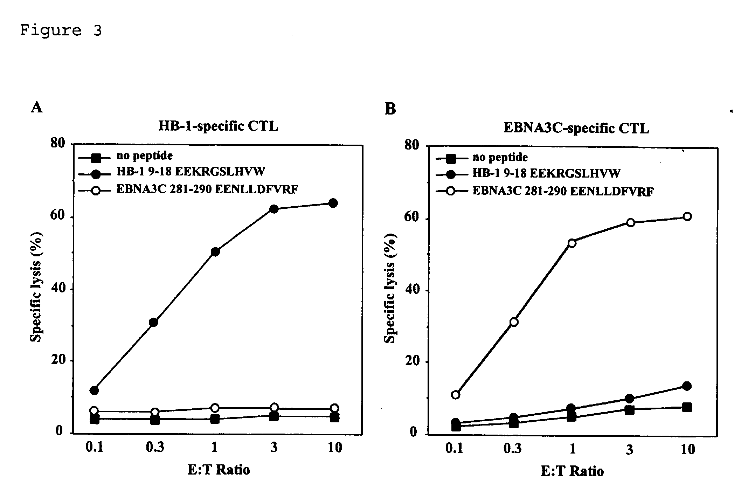

The cDNA encoding the HB-1 Ag consists of 397 nucleotides with no significant homology to sequences presently recorded in data banks. To localise the region encoding the HB-1 epitope, we transfected COS-1 cells with truncated HB-1 cDNA constructs in combination with the HLA-B44 cDNA and tested their capacity of inducing IFN-γ release by the CTL (Fig. 2). The smallest truncated construct that still encoded both the translation initiation codon and the peptide coding region contains the nucleotide sequence 100 to 165 (Fig. 2). The three possible translational reading frames within this sequence did not code for a peptide according to the described HLA-B44 binding motif, a Glu at position 2 and a Phe or Tyr at position 9 or 10 (18,19). Next, we synthesised all 9-, 10- and 11-mer peptides with a Glu residue at position 2 encoded by the translational reading frames within nucleotide sequence 100 to 165, and tested their ability to induce lysis by CTL MP1 upon loading on HB-1-negative target cells. The 10-mer EEKRGSLHVW was specifically recognised by CTL MP1, but not by HLA-B44-restricted EBNA3C specific CTL (Fig 3 *A* and *B*). CTL MP1 did not recognise the 9-mer peptide EEKRGSLHV (data not shown).

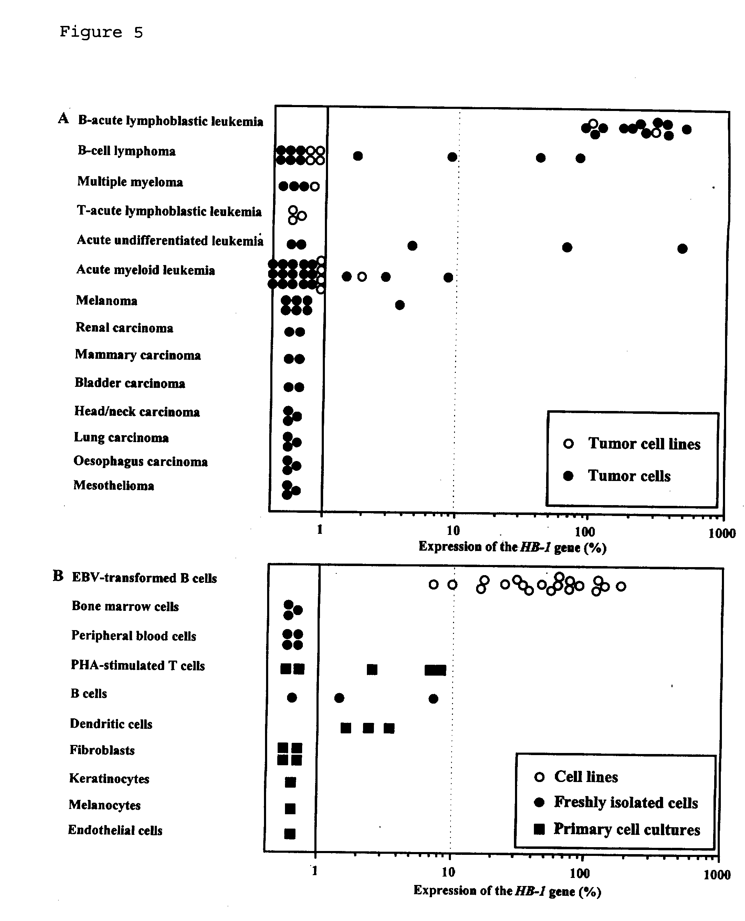

The open reading frame (ORF) encoding the EEKRGSLHVW CTL epitope contains a CTG translation initiation codon resulting in a putative 41-amino acid protein (Fig. 4). Substitution of this CTG into AAG resulted in a complete loss of the ability to stimulate IFN-γ release by CTL MP1 upon transfection into COS-1 cells (data not shown). Together, these data led us to conclude that within the *HB-1* sequence the CTG at position 108-110 initiates the translation of a 41-amino acid protein from which the EEKRGSLHVW CTL epitope is generated. *The HB-1 gene is only expressed by transformed B cells.* Interestingly, we observed that CTL MP1 exhibits specific cytotoxicity towards leukemia- and EBV-transformed B cells, but not against untransformed B cells, T cells, monocytes and fibroblasts (8). Therefore, we studied the level of *HB-1* gene expression in a large panel of tumour and non-malignant cells using real time quantitative RT-PCR. All B-ALL samples expressed the *HB-1* gene at a significant level exceeding 10% of that found in the reference B-ALL cell line KM3 (Fig. 5 *A*). In addition, two out of fourteen B-cell lymphomas and two out of five acute undifferentiated leukaemia's showed significant HB-1 expression. In contrast, all T-ALLs, multiple myelomas, acute myeloid leukaemia's, and non-hematological solid tumours lacked HB-1 expression. Since the number of HB-1 transcripts in B-ALL cells is even lower than that of the low copy gene *porphobilinogen deaminase (Pbgd)* we concluded that HB-1 is a rare mRNA species. This notion was confirmed by the observation that we could not detect HB-1 expression by Northern blot analysis, whereas β-actin mRNA was readily detected (data not shown).

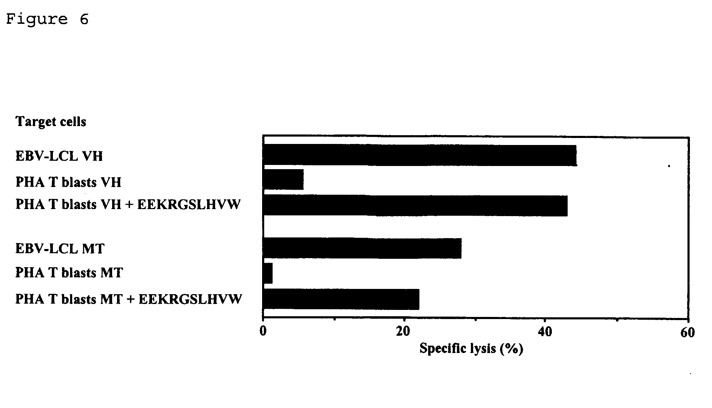

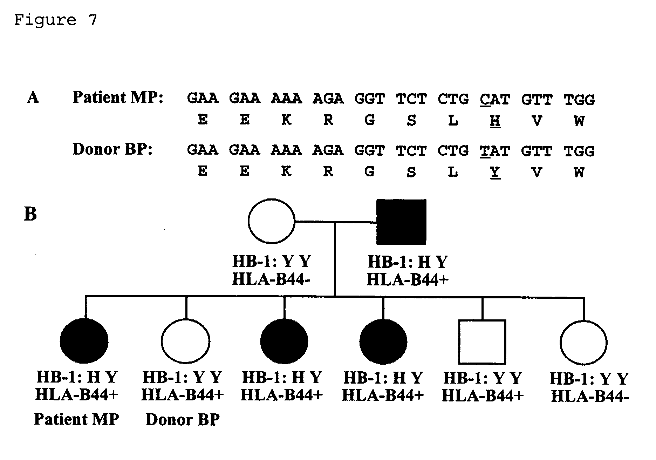

Analysis of the expression of the *HB-1 gene* in a panel of non-malignant cells revealed significant levels in 90% of the EBV-transformed B cell lines, whereas no significant HB-1 transcription was observed in all other non-malignant cell types (Fig. 5 *B*). Some B cell and PHA-stimulated T cell samples express very low levels of HB-1 (<10% of that found in the reference B-ALL cell line KM3; Fig. 5 *B*). PHA-stimulated T cell blasts with an HB-1 expression level of 8% and 2.5% of that found in the KM3 cell line were not recognised by CTL MP1 (Fig. 6). Loading of these PHA-stimulated T cell blasts with the EEKRGSLHVW peptide results in lysis that is as high as the killing of the HB-1-positive EBV-LCL. Analysis of HB-1 expression in normal tissues revealed only low (<10%) transcription levels in testis samples (Table 1). Together, these results demonstrate that substantial HB-1 mRNA expression is observed only in B-ALL cells and in EBV-transformed B cells and that very low mRNA expression of HB-1 does not result in lysis by CTL MP1. *A polymorphism in the HB-1 gene determines CTL recognition.* CTL MP1 lyse EBV-transformed B cells derived from the patient MP, whereas EBV-transformed B cells derived from the HLA-identical sibling donor BP are not recognised (8). As the *HB*-1 gene was significantly expressed by both cell lines, these results suggested the presence of a polymorphism in this sequence. Analysis of the *HB-1* sequence of the donor revealed that it differs from that of the patient at only one nucleotide (position 153: C to T), leading to an amino acid change from H to Y in the HB-1 peptide (Fig. 7 *A).* The corresponding alleles were named *HB-1*^{*H*} and *HB-1*^{*Y*}*,* respectively.

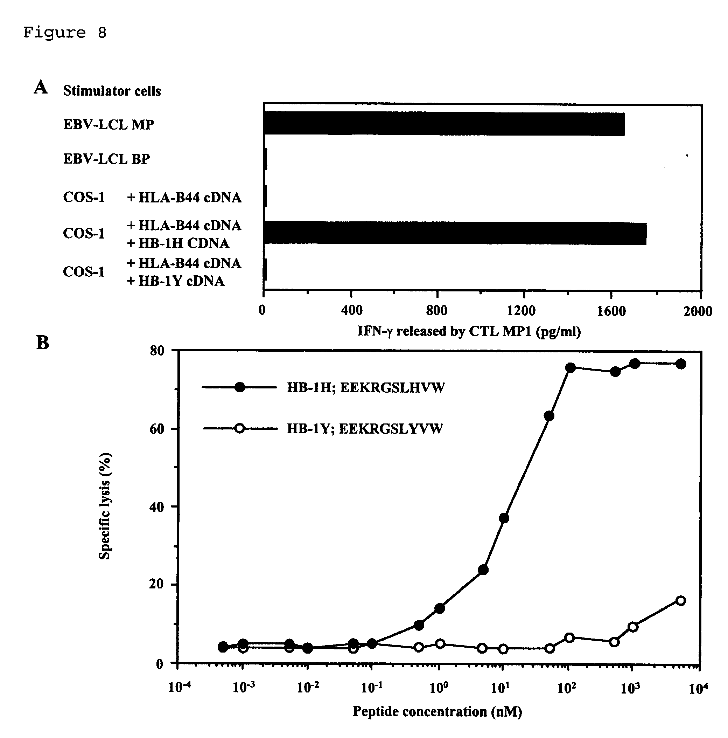

Studies of the *HB-1* gene polymorphism in relatives of patient MP resulted in a clear correlation between recognition by CTL MP1 of EBV-LCL of HLA-B44-positive family members and the expression of the *HB-1*^{*H*} allele (Fig. 7 B). To verify that only expression of the *HB-1*^{*H*} allele leads to recognition by CTL MP1, we transfected HB-1^{H} or HB-1^{Y} cDNA along with HLA-B44 cDNA into COS-1 cells. Cells transfected with the HB-1^{H} cDNA stimulated IFN-γ release by the CTL, whereas cells transfected with the HB-1^{Y} cDNA did not (Fig. 8 *A).* In addition, EBV-transformed B cells incubated with the peptide encoded by the *HB-1*^{*Y*} allele were not lysed by CTL MP1 (Fig. 8 *B).* This is likely not the result of defective binding of the HB-1^{Y} peptide to HLA-B44 molecules as both peptides appeared to bind with equal affinity to these HLA molecules (data not shown). These results demonstrate that at least two allelic forms of the *HB-1* gene exist, and that only the *HB-1*^{*H*} allele encodes the peptide that is recognised by CTL MP1.

### Discussion

Over the past few years, a large number of CTL-defined tumour Ags and their encoding genes have been identified in melanoma (20). However, little is known about CTL-defined Ags encoded by genes with expression restricted to leukemia. Using biochemical methods to isolate and sequence antigenic peptides presented by MHC class I molecules, the first CTL epitopes on leukemic cells have recently been identified (6,7,21). Expression of the identified polymorphic Ags HA-1 and HA-2 is restricted to hematopoietic cells but they are not leukemia specific (2). Although these mHag have this limited tissue distribution, mismatching for HA-1 is associated with the occurrence of severe GVHD (22). To the best of our knowledge the polymorphic *HB-1* gene product described here is the first identified leukemia-associated mHag that is only significant expressed by leukemia- and EBV-transformed B cells. In some normal cells few HB-1 transcripts are present, but expression is to low for recognition by HB-1 specific CTL, as also observed for a number of other tumour-associated Ags (20,23-25). Low levels of transcription of the melanocyte-associated Ag gp100 have been found in nearly all normal tissues and tumour cell lines of non-melanocytic origin, but no gp100 protein could be detected either by Western blot or by cytotoxicity assays (23). Similarly, the Ag encoded by the *N-acetylglucosaminyltransferase V (GnTV)* gene was not recognised by specific CTL when transcription levels did not exceed 8% of that of the reference melanoma cell line (24). Finally, MAGE-1 specific CTL are unable to recognise tumour cell lines expressing low levels (<10%) of the *MAGE-1* gene when compared with melanoma cells that were efficiently lysed (25). These reported data and the observation that PHA-stimulated T cells expressing low levels of the *HB-1* gene (2.5% and 8% of that of the reference B-ALL cell line) are not lysed by CTL MP1 indicate that only significant HB-1 transcription leads to CTL recognition.

The HB-1 antigenic peptide is encoded by a sequence which starts with a CUG codon resulting in the translation of a short protein of 41-amino acids. Such an unusual initiation of translation at a CUG has been reported previously (26). Whether this product is the only protein encoded by the *HB-1* gene or whether there are more proteins encoded by alternative ORFs is currently unknown. Although most eukaryotic mRNAs have a single ORF of which translation is usually initiated by an AUG codon, several human genes are bicistronic encoding two proteins (27-29). For instance the gp75 gene in melanoma has two overlapping ORFs resulting in two completely different proteins: 1) gp75 as recognised by IgG antibodies in the serum from melanoma patients, and 2) a short protein of 24-amino acids from which an antigenic peptide is generated recognised by CTL (27). Whether the generation of the short 41-amino acid protein from the *HB-1* gene is the result of translation of an alternative ORF awaits further characterisation of the gene.

At present we can not exclude that the *HB-1* gene encoded protein is a B cell differentiation Ag which is overexpressed in B-ALL cells, and lost in mature B cells. Alternatively, the malignant transformation of progenitor B cells itself may induce HB-1 expression. This latter possibility is supported by the finding that the *HB-1* gene also shows significant expression in B cells which are transformed *in vitro* with EBV. These EBV-transformed B cell lines express all EBV gene encoded proteins, whereas EBV-positive Burkitt's lymphoma cell lines express only the EBNA1 protein (30). Since HB-1 is significantly expressed by EBV-transformed B cell lines and not by Burkitt's lymphoma cell lines it is tempting to speculate that expression of EBV gene encoded proteins other than EBNA1 may induce HB-1 mRNA transcription in mature B cells. Studies dealing with expression levels during B-cell differentiation, and the role of EBV transformation in inducing HB-1 expression are currently under investigation. The antigenic peptide EEKRGSLHVW encoded by the *HB-1* gene is recognised in association with HLA-B44, which is a common HLA-B allele expressed by 23% of the Caucasian population. Five subtypes of HLA-B44 have been identified but the most frequently expressed subtypes are HLA-B*4402 and -B*4403. These two subtypes differ only by a single amino acid substitution from Asp (*4402) to Leu (*4403) in position 156 of the a2 domain (18,31,32). Both HLA-B*4402 and -B*4403 are able to present the HB-1 peptide to CTL clone MP1. The consensus peptide-binding motif for HLA-B44 shows a predominance for Glu at position 2, and Tyr or Phe at position 9 or 10 (18,19). The HB-1 peptide contains a Glu at position 2, but in contrast to the consensus motif it has a Trp at position 10 indicating that all amino acids with aromatic side chains facilitate binding to HLA-B44. A polymorphism in the *HB-1* gene resulted in a single amino acid exchange from His to Tyr at position 8 of the HB-1 peptide. The anchor residues of the peptide are not involved in this substitution, and both the HB-1^{H} and HB-1^{Y} peptide bind with similar affinity to HLA-B44 molecules. Since the HB-1^{Y} peptide is not recognised by CTL clone MP1 the polymorphism appears to influence a TCR contact residue.

The molecular identification and characterisation of the leukemia-associated mHag HB-1 allows the opportunity to treat leukemia patients with immunotherapy specifically targeted to the tumour without the risk of inducing GVHD. For this, patients must be typed for the presence of the HB-1^{H} allele and their tumour cells must significantly express the *HB-1* gene. The mHag HB-1 might turn out to be an excellent target against which specific CTL can easily be generated from allogeneic donors and adoptively transferred into BMT recipients with relapsed B-ALL. We have already succeeded in generating HB-1 specific CTL *in vitro* from peripheral blood of healthy HLA-B44-positive individuals by stimulating CD8-positive T cells with HB-1 peptide-loaded autologous dendritic cells. These induced CTL displayed lysis of both peptide-loaded HB-1-negative target cells, and autologous HLA-B44-positive EBV-transformed B cells endogenously expressing the HB-1 Ag. The presence of HB-1 specific CTL in the T cell repertoire of HB-1-positive individuals and the restricted expression of the *HB-1* gene by B-ALL cells may also allow the use of HB-1 encoded Ags for vaccination protocols to induce specific immunity in B-ALL patients.

### BRIEF DESCRIPTION OF THE DRAWINGS

Figure 1. Identification of the cDNA encoding the antigenic peptide recognised by CTL clone MP1. Production of IFN-γ is shown upon stimulation with the following stimulator cells: B-ALL cells, EBV-transformed B cells, and B cells stimulated with CD40 plus 100 U/ml TNF-α for 2 days of the HLA-B44, HB-1-positive patients MP and VR, and COS-1 cells cotransfected with the HB-1 cDNA plus HLA-B44 cDNA, and COS-1 cells transfected either with the HLA-B44 cDNA alone or with HB-1 cDNA alone. Release of IFN-γ was measured by ELISA.

Figure 2. Location of the nucleotide sequence coding for the translation initiation codon and the antigenic peptide recognised by CTL MP1. HB-1 cDNA deletion constructs were cloned into an expression vector and cotransfected with the HLA-B44 cDNA into COS-1 cells. Transfected cells and CTL MP1 were incubated for 18 h and the release of IFN-γ was measured by ELISA.

Figure 3. Identification of the HB-1 antigenic peptide. (A) Cytolytic activity by CTL clone MP1 against HLA-B44-positive target cells incubated with 5 µM of HB-1 peptide EEKRGSLHVW. Controls included the HLA-B44-positive target cells incubated either without peptide or with the EBNA3C 281-290 peptide EENLLDFVRF. (B) Cytolytic activity by the EBNA3C specific CTL against HLA-B44-positive target cells incubated with 5 µM of EBNA3C peptide EENLLDFVRF. Controls included the HLA-B44-positive target cells incubated either without peptide or with the HB-1 peptide EEKRGSLHVW.

Figure 4. Sequence of HB-1 cDNA and of the 41-amino acid encoded protein starting from the CTG start codon (underlined) at nucleotide positions 108 to 110. The sequence corresponding to the HLA-B44-restricted HB-1 peptide is boxed. These sequence data are available from EMBL/GenBank/DDBJ under accession number AF103884.

Figure 5. Expression of the *HB-1* gene in tumour cells and non-malignant cells. (A) Measurement of the HB-1 expression obtained by real time quantitative RT-PCR in tumour cells and cell lines. The following cell lines were used: B-ALL (KM3, BV173), B-cell lymphoma (Daudi, Raji, Ramos, SU-DHL6), multiple myeloma (RPMI1758), T-ALL (Jurkat, CEM, HSB-2), acute myeloid leukemia (Lama, Kasumi, K562, HL60, KG-1). The expression levels were determined by a calibration function generated from RNA of the HB-1-positive B-ALL cell line KM3 and expressed relative to the HB-1 level measured in these cells. The *Pbgd* gene was used as standard to correct for RNA quantity and quality. The detection limit is indicated with a solid line. Samples showed significant *HB-1* gene expression if they exceeded 10% of that found in the B-ALL cell line KM3. This arbitrary threshold is indicated with a dashed line. *(B)* Measurement of the HB-1 expression obtained by real time quantitative RT-PCR in freshly isolated cells or primary cell cultures.

Figure 6. Cytolytic activity by CTL clone MP1 against EBV-LCL and PHA-stimulated T cell blasts of HLA-B44, HB-1-positive individuals. PHA-stimulated T cell blasts were pre-incubated either without peptide or with the HB-1 peptide EEKRGSLHVW. The level of *HB-1* gene expression in EBV-LCL VH and MT was respectively 140% and 122% of that found in the B-ALL cell line KM3, and in PHA-stimulated T cell blasts VH and MT 8% and 2.5%, respectively. Both individuals express homozygous the HB-1^{H} allele and the HLA-B44 subtype of VH is B*4403 and of MT B*4402. The E:T cell ratio was 3:1.

Figure 7. The mHag HB-1 is encoded by the HB-1^{H} allele of the *HB-1* gene. (A) Sequence of the peptide coding region of the *HB-1* gene of patient MP and donor BP. The nucleotide and amino acid polymorphism are underlined. (B) Correlation between expression of HB-1 alleles and cytolytic activity by CTL clone MP1 against EBV-transformed B cell lines of relatives of patient MP. Filled circles (females) or squares (males) indicate strong lysis by CTL MP1. Open symbols indicate no lysis. Expression of HB-1 alleles was determined by RT-PCR amplification and digestion of PCR products with restriction enzyme NIaIII.

Figure 8. The single amino acid exchange within the HB-1 antigenic peptide is critical for recognition by CTL clone MP1. (A) Production of IFN-γ is shown upon stimulation with EBV-transformed B cells of patient MP and donor BP, and COS-1 cells cotransfected with the HB-1^{H} or HB-1^{Y} cDNA and HLA-B44 cDNA. (B) Cytolytic activity by CTL clone MP1 against HLA-B44-positive target cells incubated with the HB-1^{H} or HB-1^{Y} peptide. The E:T cell ratio was 10:1.

### References

1. Horowitz, M.M., R.P. Gale, P.M. Sondel, J.M. Goldman, J. Kersey, H.J. Kolb, A.A. Rimm, O. Ringden, C. Rozman, B. Speck, R.L. Truitt, F.E. Zwaan, and M.M. Bortin. 1990. Graft-versus-leukemia reactions after bone marrow transplantation. *Blood* 75:555-562.
2. Goulmy, E. 1996. Human minor histocompatibility antigens. *Curr. Opin. Immunol.* 8:75-81.
3. Perrault, C., D.C. Roy, and C. Fortin. 1998. Immunodominant minor histocompatibility antigens: the major ones. *Immunol. Today* 19:69-74.
4. Pardoll, D. 1997. Taming the sinister side of BMT: Dr. Jekyll and Mr. Hyde. *Nature Med.* 3:833-834.
5. Wallny, H.J., and H.G. Rammensee. 1990. Identification of classical minor histocompatibility antigen as cell-derived peptide. *Nature* 343:275-278.
6. Wang, W., L.R. Meadows, J.M.M. den Haan, N.E. Sherman, Y. Chen, E. Blokland, J. Shabanowitz, A.I. Agulnik, R.C. Hendrickson, C.E. Bishop, D.F. Hunt, E. Goulmy, and V.H. Engelhard. 1995. Human H-Y: a male-specific histocompatibility antigen derived from the SMCY protein. *Science* 269:1588-1590.
7. Den Haan, J.M.M., L.M. Meadows, W. Wang, J. Pool, E. Blokland, T.L. Bishop, C. Reinhardus, J. Shabanowitz, R. Offringa, D.F. Hunt, V.H. Engelhard, and E. Goulmy. 1998. The minor histocompatibility antigen HA-1: a diallelic gene with a single amino acid polymorphism. *Science* 279:1054-1057.
8. Niederwieser, D., A. Grassegger, J. Aubock, M. Herold, D. Nachbaur, A. Rosenmayr, A. Gachter, W. Nussbaumer, S. Gaggl, M. Ritter, and C. Huber. 1993. Correlation of minor histocompatibility antigen-specific cytotoxic T lymphocytes with graft-versus-host disease status and analysis of tissue distribution of their target antigens. *Blood.* 81:2200-2208.
9. Warren, E.H., P.D. Greenberg, and S.R. Ridell. 1998. Cytotoxic T-lymphocyte-defined human minor histocompatibility antigens with a restricted tissue distribution. *Blood* 91:2197-2207.
10. Faber, L.M., S.A.P. van Luxemburg-Heijs, R. Willemze, and J.H.F. Falkenburg. 1992. Generation of leukemia-reactive cytotoxic T lymphocyte clones from the HLA-identical bone marrow donor of a patient with leukemia. *J. Exp. Med.* 176:1283-1289.
11. Dolstra, H., H. Fredrix, F. Preijers, E. Goulmy, C.G. Figdor, T.M. de Witte, and E. van de Wiel-van Kemenade. 1997. Recognition of a B cell leukemia-associated minor histocompatibility antigen by CTL. *J. Immunol.* 158:560-565.
12. Ennis, P.D., J. Zemmour, R.D. Salter, and P. Parham. 1990. Rapid cloning of HLA-A,B cDNA by using the polymerase chain reaction: frequency and nature of errors produced in amplification. *Proc. Natl. Acad. Sci. USA* 87:2833-2837.
13. Van de Wiel-van Kemenade, E., A.A. te Velde, A.J. de Boer, R.S. Weening, A. Fischer, J. Borst, C.J.M. Melief, and C.G. Figdor. 1992. Both LFA-1-positive and -deficient T cell clones require the CD2/LFA-3 interaction for specific cytolytic activation. *Eur. J. Immunol.* 22:1467-1475.
14. Higuchi, R., C. Fockler, G. Dollinger, and R. Watson. 1993. Kinetic PCR analysis: real-time monitoring of DNA amplification reactions. *Biotechnology* 11:1026-1030.
15. Gibson, U.E.M., C.A. Heid, and P.M. Williams. 1996. A novel method for real time quantitative RT-PCR. *Genome Res.* 6:995-1001.
16. Chretien, S., A. Dubart, D. Beaupain, N. Raich, B. Grandchamp, J. Rosa, M. Goossens, and P.H. Romeo. 1988. Alternative transcription and splicing of the human porphobilinogen deaminase gene result either in tissue-specific or in housekeeping expression. *Proc. Natl. Acad. Sci. USA* 85:6-10.
17. Finke, J., R. Fritzen, P. Ternes, W. Lange, and G. Dolken. 1993. An improved strategy and a useful housekeeping gene for RNA analysis from formaline-fixed, paraffin embedded tissues by PCR. *Biotechniques* 14:448-453.
18. Fleischhauer, K., D. Avila, F. Vilbois, C. Traversari, C. Bordignon, and H.J. Wallny. 1994. Characterisation of natural peptide ligands for HLA-B*4402 and -B*4403: implications for peptide involvement in allorecognition of a single amino acid change in the HLA-B44 heavy chain. *Tissue Antigens* 44:311-317.
19. DiBrino, M., K.C. Parker, D.H. Margulies, J. Shiloach, R.V. Turner, W.E. Biddison, and J.E. Coligan. 1995. Identification of the peptide binding motif for HLA-B44, one of the most common HLA-B alleles in the Caucasion population. *Biochemistry* 34:10130-10138.
20. Van den Eynde, B.J., and P. van der Bruggen. 1997. T cell defined tumour antigens. *Curr. Opin. Immunol.* 9:684-693.
21. Den Haan, J.M.M., N.E. Sherman, E. Blokland, E. Huczko, F. Koning, J.W. Drijfhout, J. Skipper, J. Shabanowitz, D.F. Hunt, V.H. Engelhard, and E. Goulmy. 1995. Identification of a graft versus host disease-associated human minor histocompatibility antigen. *Science* 268:1476-1480.
22. Goulmy, E., R. Schipper, J. Pool, E. Blokland, J.H. Falkenburg, J. Vossen, A. Grathwohl, G.B. Vogelsang, H.C. van Houwelingen, and J.J. Rood. 1996. Mismatches of minor histocompatibility antigens between HLA-identical donors and recipients and the development of graft-versus-host disease after bone marrow transplantation. *N. Engl. J. Med.* 334:281-285.
23. Brouwenstijn, N., E.H. Slager, A.B.H. Bakker, M.W.J. Schreurs, C.W. van der Spek, G.J. Adema, P.I. Schrier, and C.G. Figdor. 1997. Transcription of the gene encoding melanoma-associated antigen gp100 in tissues and cell lines other than those of the melanocytic lineage. Br. *J. Cancer* 76:1562-1566.
24. Guilloux, Y., S. Lucas, V.G. Brichard, A. van Pel, C. Viret, E. de Plaen, F. Brasseur, B. Lethe, F. Jotereau, and T. Boon. 1996. A peptide recognised by human cytolytic T lymphocytes on HLA-A2 melanomas is encoded by an intron sequence of the N-acetylglucosaminyltransferase V gene. J. *Exp. Med.* 183:1173-1183.
25. Lethe, B., P. van der Bruggen, F. Brasseur, and T. Boon. 1997. MAGE-1 expression threshold for the lysis of melanoma cell lines by a specific CTL. *Melanoma Res.* 7(suppl 2):S83-S88.
26. Hann, S.R. 1994. Regulation and function of non-AUG-initiated proto-oncogenes. *Biochemie* 76:880-886.
27. Descombes, P., and U. Schibler. 1991. A liver-enriched transriptional activator protein, LAP, and a transcriptional inhibitory protein, LIP, are translated from the same mRNA. *Cell* 67:569-579.
28. Wang, R.F., M.R. Parkhurst, Y. Kawakami, P.F. Robbins, and S.A. Rosenberg. 1996. Utilization of an alternative open reading frame of a normal gene in generating a novel human cancer antigen. *J. Exp. Med.* 183:1131-1140.
29. Shichijo, S., M. Nakao, Y. Imai, H. Takasu, M. Kawamoto, F. Niiya, D. Yang, Y. Toh, H. Yamana, and K. Itoh. 1998. A gene encoding antigenic peptides of human squamous cell carcinoma recognised by cytotoxic T lymphocytes. *J. Exp. Med.* 3:277-288.
30. Klein, G. 1994. Epstein-Barr virus strategy in normal and neoplastic B cells. *Cell* 77:791-793.
31. Tiercy, J.M., N. Djavad, N. Rufer, D.E. Speiser, M. Jeannet, and E. Roosnek. 1994. Oligotyping of HLA-A2, -A3, and -B44 subtypes: Detection of subtype incompatibilities between patients and their serologically matched unrelated bone marrow donors. *Hum. Immunol.* 41:207-215.
32. Fleischhauer, K., N.A. Kernan, B. Dupont, and S.Y. Yang. 1991. The two major subtypes of HLA-B44 differ for a single amino acid at codon 156. *Tissue antigens.* 37:133-137.

**Table 1.**

| Expression of the HB-1 gene in normal tissues. | | | |
|---|---|---|---|
| Type of samples/ normal tissue tested^{a} | positive samples/ samples tested^{a} | Type of normal tissue | positive samples |
| Adrenal gland | 0/2 | Lung | 0/3 |
| Brain | 0/1 | Muscle | 0/2 |
| Breast | 0/2 | Ovary | 0/2 |
| Cerebellum | 0/1 | Prostate | 0/1 |
| Colon | 0/4 | Rectum | 0/3 |
| Endometrium | 0/3 | Retina | 0/3 |
| Heart | 0/2 | Skin | 0/6 |
| Ileum | 0/1 | Testis | 3/3 (<10%) |
| Kidney | 0/3 | Urinary bladder | 0/3 |
| Liver | 0/4 | Uterus | 0/3 |

| | | | |
|---|---|---|---|
| ^{a}Expression of the *HB-1* gene was determined by real time quantitative RT-PCR of total RNA as described in Materials and Methods. Samples were scored as positive if their *HB-1* gene expression exceeded 1% of that found in the B-ALL cell line KM3. | | | |

## Claims

1. A tumour specific antigen or an immunogenic part, ^{'} derivative or analogue of said antigen, which is a minor histocompatibility antigen.

2. An antigen according to claim 1, wherein said antigen is essentially specific for leukemia or lymphoma, preferably B-cell leukemia, more preferably B cell acute lymphoblastic leukemia.

3. An antigen according to claim 1 or claim 2, wherein said antigen is a polymorphic antigen.

4. An antigen according to anyone of claims 1-3, wherein said antigen is a human antigen.

5. An antigen according to anyone of claims 1-4, wherein said antigen comprises an amino-acid sequence as depicted in figure 4 or an immunogenic part, derivative or analogue thereof.

6. A peptide comprising an immunogenic part of an antigen according to anyone of claims 1-5.

7. A peptide according to claim 6, wherein said peptide is capable of associating with at least one major histocompatibility complex I molecule or major histocompatibility complex II molecule or both.

8. A peptide according to claim 6 or claim 7, comprising the amino-acid sequence EEKRGSLHVW and/or the amino-acid sequence EEKRGSLYVW, or a functional part, derivative or analogue thereof.

9. A nucleic acid encoding an antigen according to anyone of claims 1-5 or a peptide according to anyone of claims 6-8.

10. A nucleic acid according to claim 9, wherein said nucleic acid comprises a nucleic acid sequence depicted in figure 4, or a functional part, derivative or analogue thereof.

11. A nucleic acid delivery vehicle comprising at least a nucleic acid according to claim 9 or claim 10.

12. A nucleic acid delivery vehicle according to claim 11, further comprising a nucleic acid encoding at least one protein capable of modulating an immune response.

13. A nucleic acid delivery vehicle according to claim 11 or claim 12, wherein said protein is a co-stimulatory protein, an immune response inhibitory protein, an interleukin, a major histocompatibility complex I protein or a major histocompatibility complex II protein.

14. A nucleic acid delivery vehicle according to anyone of claims 11-13, wherein said vehicle has at least in part been provided with a tissue tropism for smooth muscle cells, hemopoietic stem cells, antigen presenting cells, endothelial cells and/or their progenitors.

15. A nucleic acid delivery vehicle according to anyone of claims 11-14, wherein said vehicle has at least in part been deprived of tissue tropism for liver cells.

16. A nucleic acid delivery vehicle according to claim 14 or claim 15, wherein said changed tissue tropism is provided by at least a tissue tropism determining part of a fiber protein of a group B adenovirus, preferably adenovirus 16.

17. A nucleic acid delivery vehicle according to anyone of claims 11-16, wherein said nucleic acid further comprises integration means of an adeno-associated virus nucleic acid.

18. A nucleic acid delivery vehicle according to anyone of claims 11-17, wherein said vehicle is an adenovirus particle, an adeno-associated virus particle or a retrovirus particle.

19. An adenovirus particle which is a gene delivery vehicle according to claim 18, wherein said nucleic acid further comprises an adenovirus vector and wherein said vector is at least in part deprived of the capability to express E1-region encoded proteins, preferably through a deletion in the El-region.

20. An adenovirus particle according to claim 19, wherein said vector is further at least in part deprived of the capability to express E2A protein.

21. An adenovirus particle according to claim 19 or claim 20, wherein said vector is further at least in part deprived of the capability to express at least one of the E3-region encoded proteins.

22. An adenovirus particle according to anyone of claims 11-21, wherein said vector is further at least in part deprived of the capability to express at least one of the E4-region encoded proteins, preferably E4-Orf3 and/or E4-Orf6.

23. A cell for the production of an adenovirus particle according to anyone of claims 19-22, comprising one or more means for the complementation of at least part of the function of one or more of the proteins, the capability of expression of which said vector has at least in part been deprived.

24. A cell according to claim 23, wherein said cell is derived from a PER.C6 cell (ECACC deposit number 96022940).

25. A method for generating, promoting or modulating an immune response against tumour cells in an individual, comprising administering to said individual a composition comprising at least one antigen according to anyone of claims 1-5, at least one peptide according to anyone of claims 6-8, at least one nucleic acid according to claim 9 or claim 10, at least one nucleic acid delivery vehicle according to anyone of claims 11-18, or at least one adenovirus particle according to anyone of claims 19-22, or a combination thereof.

26. A method according to claim 25, wherein said tumour cells are leukemic cells, preferably leukemic B cells, more preferably acute lymphoblastic leukemic B cells.

27. A method for generating tumour specific T lymphocytes, comprising culturing CD8 positive T cells together with target cells, wherein said target cells have been provided with at least one peptide according to anyone of claims 6-8.

28. A method according to claim 27, wherein said T cells and said target cells are derived from essentially the same individual.

29. Tumour-specific T lymphocytes obtainable by a method according to anyone of claims 25-28.

30. T-lymphocytes according to claim 29, provided with a nucleic acid encoding a herpes simplex virus thymidine kinase.

31. T-lymphocytes according to claim 29 or claim 30, provided with a nucleic acid encoding at least one protein capable of stimulating an immune response, such as a co-stimulatory protein or an interleukin.

32. A method for the treatment of an individual suffering from or at risk of suffering from a tumour comprising administering to said individual T-lymphocytes according to anyone of claims 29-31.

33. Use of T lymphocytes according to anyone of claims 29-31 in a medicament.

34. Use of a minor histocompatibility antigen or functional part, derivative or analogue thereof, essentially specific for tumour cells for the generation of tumour specific T lymphocytes.

35. An antibody with a specificity for an antigen according to anyone of claims 1-5 or a peptide according to anyone of claims 6-8.

36. An antibody capable of recognising a peptide according to anyone of claims 6-8 in the context of MHC-I or MHC-II.

37. Use of an antibody according to claim 35 or claim 36, in an immunoassay for the detection of tumour cells.

38. A vaccine comprising at least an antibody according to claim 35 or claim 36, and a pharmaceutically acceptable carrier or diluent.

39. A method for the identification of cells comprising detecting RNA encoding an antigen according anyone of claims 1-5 in a sample comprising RNA of said cells.

40. A method for the identification of cells using an antibody according to claim 35 or claim 36.

41. A kit for the detection of cells comprising at least one antibody according to claim 35 or claim 36.

42. A kit for the detection of cells comprising at least two primers and a probe directed to a nucleic acid according to claim 9 or claim 10.

43. A cloning vehicle comprising a nucleic acid according to claim 9 or claim 10.

44. A cell having been provided with a nucleic acid according to claim 9 or claim 10.

45. A vaccine comprising antigen presenting cells, containing MHC class I, or MHC class II molecules or both, with a peptide according to anyone of claims 6-8.

46. A vaccine according to claim 45, wherein said cells are further provided with a nucleic acid by a gene delivery vehicle according to anyone of claims 11-18, or an adenovirus particle according to anyone of claims 19-22.

47. A vaccine comprising a gene delivery vehicle according to anyone of claims 11-18, or an adenovirus particle according to anyone of claims 19-22.

48. Use of at least one antigen according to anyone of claims 1-5, at least one peptide according to anyone of claims 6-8, at least one nucleic acid according to claim 9 or claim 10, at least one nucleic acid delivery vehicle according to anyone of claims 11-18, or at least one denovirus particle according to anyone of claims 19-22, or a combination thereof in a medicament.

49. T lymphocytes capable of recognising cells presenting peptides in the context of MHC class I or class II, encoded by a HB-1 gene, or a functional part, derivative or analogue thereof.

50. A method for detecting T lymphocytes according to claim 49, comprising loading labelled tetrameric MHC-I and/or MHC-II molecules with a peptide according to anyone of claims 6-8, contacting said T lymphocytes with said peptide loaded MHC molecules and detecting said label.
